# EUROPÄISCHE PATENTANMELDUNG

(11) **EP 1 293 219 A1**
(43) Veröffentlichungstag der Anmeldung: **19.03.2003**
(21) Anmeldenummer: 01122251.0
(22) Anmeldetag: 17.09.2001
(51) Int. Cl.: A61L 27/32, A61L 31/08

(54) **Biomedizinisches Erzeugnis mit einem eine Nickel-Titan-Legierung enthaltenden Substrat und einer Calciumphosphat enthaltenden überdeckenden Schicht**

(71) Anmelder: Epple, Matthias, Prof. Dr., 45529 Hattingen (DE); Choi, Jongsik, Dr., 44229 Dortmund (DE); Müller, Dietmar, Dr., 44799 Bochum (DE)
(72) Erfinder: Epple, Matthias, Prof. Dr., 45529 Hattingen (DE); Choi, Jongsik, Dr., 44229 Dortmund (DE); Müller, Dietmar, Dr., 44799 Bochum (DE)
(74) Vertreter: Siemons, Norbert, Dr.-Ing.

(57) **Zusammenfassung**

Erzeugnis für die Biomedizin mit einem eine Nickel-Titanlegierung enthaltenden Substrat und mindestens einer, durch Eintauchen in Calciumphosphat-Lösung auf das Substrat aufgebrachten, mindestens ein Calciumphosphat enthaltenden Schicht.

## Beschreibung

Die Erfindung bezieht sich auf ein Erzeugnis für die Biomedizin mit einem eine Nickel-Titan-Legierung enthaltenden Substrat und einer dieses überdeckenden Schicht und auf ein Verfahren, das zur Herstellung des Erzeugnisses geeignet ist.

### Grundlagen zu Nickel-Titan-Legierungen

Die hier angesprochenen Nickel-Titan-Legierungen sind insbesondere - aber nicht ausschließlich - Formgedächtnislegierungen auf Nickel-Titan-Basis.

Formgedächtnislegierungen weisen besondere Eigenschaften auf, die für Anwendungen in unterschiedlichen Gebieten (Konstruktionstechnik, Aktorik/Sensorik, Biomedizin) von großem Interesse sind. Dabei sind insbesondere zu nennen
(1) der Formgedächtniseffekt: Das Material kann kalt verformt werden und "erinnert" sich beim gelinden Erwärmen wieder an seine ursprüngliche Form, d.h. es nimmt die ursprüngliche Geometrie wieder ein (sogenannter "Einweg-Effekt"). Der Einweg-Effekt ist irreversibel. Durch geeignete mechanische Vorbehandlung ist es möglich, auch beim Abkühlen wieder in die erste Geometrie zurück zu kommen, d.h. ein "Schalten" zwischen zwei Geometrien ist durch thermisches Schalten möglich (sogenannter "Zweiweg-Effekt").
(2) die Superelastizität: Das Material weist eine außergewöhnliche Elastizität und Biegsamkeit auf, die von gewöhnlichem Metallen und Legierungen nicht erreicht wird. Hierfür wird z.B. in der Biomedizin bei hochelastischen Kathetern für die minimalinvasive Chirurgie Gebrauch gemacht.
(3) die Pseudoelastizität: Im Spannungs-Dehnungs-Diagramm weist das Material ein Plateau auf (einige % Dehnung), d.h. über einen größeren Dehnungsbereich bleibt die Spannung konstant. Dies unterscheidet das Material von herkömmlichen Metallen, die in der Regel einen quasi-linearen Anstieg in der Spannungs-Dehnungskurve zeigen. Diese konstante Rückstellkraft bei variabler Dehnung wird z.B. in orthodontischen Drähten (Zahnspangen) zur Korrektur von Zahnfehlstellungen ausgenutzt.

Obwohl es eine Reihe von Formgedächtnislegierungen mit unterschiedlicher Zusammensetzung gibt, ist der bei weitem prominenteste Vertreter das "Nitinol", d.h. eine Nickel-Titan-Legierung der nahezu stöchiometrischen Zusammensetzung NiTi. Durch Variation des Nickel-Titan-Verhältnisses lassen sich die mechanischen und thermischen Eigenschaften (s.o.) gezielt einstellen.

Eine der wesentlichen Anwendungen von Nitinol-Formgedächtnislegierungen liegt in der Biomedizin. Die derzeitigen Anwendungen in Deutschland für Implantate umfassen intravaskuläre Stents in der Kardiomedizin, orthodontische Drähte und Federn in der Kieferorthopädie und Klammern in der Fußchirurgie. Im Fall der Fußchirurgie beruht der vorteilhafte Effekt auf dem Einbringen der Klammer im expandierten Zustand (großer Öffnungswinkel), gefolgt von kurzzeitigem Erwärmen, welches zur Kontraktion führt. Diese Kontraktion bewirkt ein Zusammenpressen der Knochenfraktur, die die Heilung und das Zusammenwachstum begünstigt.

Ein für die biomedizinische Anwendung nachteiliger Aspekt ist der hohe Nickel-Gehalt dieser Werkstoffe, der möglicherweise zu allergenen Wirkungen führt. Die Situation wird derzeit kontrovers diskutiert, wobei das Ausmaß einer möglichen allergenen Wirkung und einer Zellschädigung kontrovers diskutiert wird:
- (Knochen-)Einwachsverhalten *in vitro* und *in vivo* vergleichbar mit chirurgischem Stahl (auch Ni-haltig).^{[1-3]}
- Keine Sensibilisierung auf der Haut.^{[3]}
- Keine Genotoxizität, aber ungünstiger als Reintitan.^{[4]}
- Gutes Knocheneinwachsverhalten in poröses Nitinol.^{[5,6]}
- Hinweise auf Induktion von Interleukinen und Cytokinen (IL-1 β^{[7]}; IL-6, TNF-α; ^{[8]}), die zu Entzündungen führen können.
- Osteosyntheseschrauben aus Nitinol und aus Stahl (14 % Ni-Gehalt) sind vergleichbar. Lokal und systemisch erhöhte Nickelkonzentrationen.^{[9]}
- Negative Effekte von Korrosionsprodukten auf Glatte Muskelzellen (Inhibierung des Wachstums ab 9 ppm Nickelionen-Konzentration).^{[10]}

In jedem Fall ist die mögliche Freisetzung von Nickel als generell negativ zu betrachten, wobei der Effekt bei Langzeit-Implantaten (wie Knochenschrauben oder Stents) kritischer zu betrachten ist als bei Kurzzeit-Implantaten (wie Zahnspangen). Die klinische Erfahrung zeigt, dass speziell bei Patienten mit bereits vorliegender Nickel-Allergie negative Auswirkungen zu befürchten sind. Die allergene Wirkung im Fall der Langzeit-Implantation (über Jahre oder Jahrzehnte) ist nicht bekannt. Der weitergehende Einsatz von Nitinol in der Biomedizin wird durch Bedenken hinsichtlich der Nickelfreisetzung stark eingeschränkt.

Davon ausgehend liegt der Erfindung die Aufgabe zugrunde, ein eine Nickel-Titan-Legierung enthaltendes Erzeugnis mit einer besseren Eignung für den Einsatz in der Biomedizin zur Verfügung zu stellen. Ferner liegt der Erfindung die Aufgabe zugrunde, ein Verfahren zur Verfügung zu stellen, das zur Herstellung des Erzeugnisses geeignet ist.

Die Aufgabe wird durch ein Erzeugnis mit den Merkmalen des Anspruches 1 und durch ein Verfahren mit den Merkmalen des Anspruches 12 gelöst. Vorteilhafte Ausgestaltungen der Erfindung sind in den Unteransprüchen angegeben.

Das erfindungsgemäße Erzeugnis für die Biomedizin weist ein eine Nickel-Titan-Legierung enthaltendes Substrat und mindestens eine, durch Eintauchen in eine Calciumphosphat-Lösung auf das Substrat aufgebrachte, mindestens Calciumphosphat enthaltende Schicht auf. Bei dem erfindungsgemäßen Verfahren wird durch Eintauchen eines eine Nickel-Titan-Legierung umfassenden Substrats in eine Calciumphosphat-Lösung mindestens eine, mindestens ein Calciumphosphat umfassende Schicht auf dem Substrat aufgebracht.

Eine Calciumphosphat-Lösung ist eine Calcium- und Phosphat-Ionen enthaltende, übersättigte wäßrige Lösung. Das mindestens eine Calciumphosphat, das in der Schicht enthalten ist, kann unterschiedlicher Beschaffenheit sein. Chemisch kann eine größere Zahl von Calciumphosphaten unterschieden werden, die sich in ihrer Elementenzusammensetzung und auch in ihren chemischen, physikalischen und biologischen Eigenschaften unterscheiden. Als Beispiele seien hier genannt das Tricalciumphosphat (TCP): Ca₃(PO₄)₂, und der Hydroxylapatit (HAP): Ca₅(PO₄)₃OH. Bei der Synthese von Calciumphosphaten (z.B. in Beschichtungen) wird fast immer ein Gemisch verschiedener Phasen erhalten. Insofern steht der Ausdruck "Calciumphosphat" fast immer für ein inniges Gemisch verschiedener Kristallphasen, von denen jede der Klasse der Calciumphosphate zuzuordnen ist. Im Rahmen der Erfindung können alle bekannten Erscheinungsformen von Calciumphosphaten auftreten.

Das Aufbringen der Schicht durch Eintauchen in Calciumphosphat-Lösung führt dazu, daß die Schicht eine wohlgeordnete "säulenartige" Struktur aufweist, die insbesondere für das Bewältigen der Volumenänderung des Nitinols beim Durchlaufen der Phasenumwandlung gut geeignet ist. Chemisch betrachtet ist die Natur der Beschichtung je nach Prozeßbedingungen einheitlich Octacalciumphosphat (OCP) oder Hydroxylapatit (HAP). Durch den Einbau von Carbonat wird eine knochenähnliche "biomimetische" Schicht aus Carbonatapatit erreicht. Carbonatapatit besteht chemisch aus Hydroxylapatid mit eingelagerten Carbonat-Ionen, wie im Knochen- und Zahnmaterial.

Bei dem erfindungsgemäß beschichteten Erzeugnis ist das Nickel und Titan enthaltende Substrat mit einer Calciumphosphat enthaltenden Schicht überdeckt, die das im Substrat enthaltende Nickel zurückhält und dessen Freisetzung verhindert. Das Calciumphosphat dient als Senke für möglicherweise frei werdendes Nickel, das als Fremdion in die Calciumphosphat-Struktur eingebaut werden kann. Calciumphosphat ist ein anerkannt biokompatibles Material, das die Mineralkomponente von Knochen und Zähnen bildet. Das erfindungsgemäß beschichtete Erzeugnis weist erheblich verbesserte biomedizinische Eigenschaften auf.

Nach einer Ausgestaltung ist das Substrat vor dem Aufbringen der Schicht chemisch vorbehandelt, um die Anlagerung des Calciumphosphats an dem Substrat zu verbessern. Hierzu kann das Substrat angeätzt und/oder oxidiert sein. Ferner kann hierzu das Substrat eine Deckschicht aus Oxiden aufweisen, die insbesondere die Komponenten TiO₂ und/oder CaO und/oder K₂O enthält (z.B. als Mischkristall, d.h. Ionenverbindung, "Titanate"). Diese Deckschichten können durch Ätzung (durch eine Base) und durch Oxidation (durch H₂O₂) herbeigeführt werden. Diese Ätzung gewährleistet den guten mechanischen Übergang von der Nickel-Titan-Legierung (insbesondere Nitinol) zum keramischen Calciumphosphat.

Nach verschiedenen Ausgestaltungen werden zusätzliche Komponenten in die Schicht eingebracht. Nach einer Ausgestaltung werden weitere anorganische Ionen eingebracht, die im Blutplasma oder im Speichel enthalten sind, z.B. CO₃²⁻ oder Mg²⁺. Nach einer weiteren Ausgestaltung werden biologisch aktive Komponenten eingebracht, z.B. Proteine oder Antibiotika. Nach einer weiteren Ausgestaltung werden organische Verbindungen zur Steuerung der Morphologie des abgeschiedenen Calciumphosphats eingebracht, z.B. Polymere, Aminosäuren, organische Salze oder Amine. Die vorerwähnten zusätzlichen Komponenten können auch in beliebiger Kombination miteinander zusätzlich in die Schicht eingebracht werden.

Nach einer weiteren Ausgestaltung wird die Beschaffenheit der Schicht durch Variation der Temperatur eingestellt, beispielsweise die Dicke oder die Verdichtung der Schicht.

### Bekannte Beschichtungen von Metalloberflächen

Für künstliche Hüftgelenke und Zahnimplantate ist eine durch Hochtemperatur-Plasmaspritzen vorgenommene Beschichtung von Titanoberflächen mit Calciumphosphaten seit Jahren Stand der Technik, um das Anwachsen von Knochen zu befördern.

Nachteilig am Plasmaspray-Verfahren sind
- hohe Investitionskosten
- unklare chemische Zusammensetzung der Beschichtung durch schnelles Abschrecken des geschmolzenen Calciumphosphats
- schwierige bis unmögliche Beschichtung innerer Oberflächen
- keine Möglichkeit zum Einbringen von biologisch aktiven Wirkstoffen
- thermische Belastung des Substrats. Dies ist besonders im Fall von Formgedächtnislegierungen kritisch, da der Formgedächtniseffekt auf einer thermisch induzierten Phasenumwandlung im Zusammenspiel mit der Metallgitter-Mikrostruktur beruht.

Ein Plasmaspray-beschichtetes Nitinol ist aus der Druckschrift Filip, P., Kneissl, A.C., Mazanev, K. "Physis of hydroxyapatite plasma coatings on TiNi shape memory materials", Mat. Sci. Eng. A234-236 (1997) 422-425 bekannt. Das Plasmaspray-Verfahren zur Beschichtung von Nitinol führt zu einer porösen, morphologisch schlecht definierten Schicht aus erstarrten, vormals geschmolzenen Partikeln. Hierdurch kann nicht die knochenähnliche "biomimetische" Schicht aus Carbonatapatit erreicht werden, wie in dem Eintauchverfahren, das erfindungsgemäß zum Einsatz kommt.

Die Beschichtung von Metalloberflächen (vor allem Titan) durch Tauchen in übersättigte Calciumphosphat-Lösungen wird seit einigen Jahren durchgeführt, ebenfalls, um das Anwachsen von Knochen zu befördern.

Die Beschichtung von Nickel-Titan-Legierungen, insbesondere von Nitinol-Legierungen, mit Calciumphosphat durch Tauchverfahren ist im Stand der Technik nicht bekannt geworden.

### Patentveröffentlichungen zur Calciumphosphat-Beschichtung von Implantaten aus wässriger Lösung.

Für Tauchbeschichtungen von Biomaterialien mit Calciumphosphat bestehen eine Reihe von Patentveröffentlichungen:
1. K. Saita and S. Fujiwara, EP 0 322 250 (eingereicht 22.12.88), "*Coating liquor containing hydroxyapatite and method for forming hydroxyapatite coating film using the same*". Dieses Verfahren beschreibt die Beschichtung nicht näher eingeschränkter Substrate (im Beispiel Titan, Aluminiumoxid-Keramik) mit einer wässrigen Dispersion aus Hydroxylapatit. Typische Schichtdicke 0.3 µm.
2. T. Kokubo, EP 0 678 300 A1 (eingereicht 07.11.94): *"Bone substitute material and process for producing the same*". Dieses Verfahren beschreibt die Beschichtung von Titan und Titanlegierungen (Ti6A14V, Ti5A12.5Sn, Ti3Al3V11Cr, Ti15Mo5Nb3Ta, Ti6A12MoTa) aus wässriger Calciumphosphat-Lösung nach Vorbehandlung mit NaOH/KOH und Erhitzen auf 400-800°C. Die Schichtdicke beträgt 5-10 µm nach drei Wochen.
3. T. Kokubo et al., EP 0 389 713 A1 (eingereicht 10.11.89): *"Process for coating bioactive hydroxyapatite film".* Dieses Verfahren erstreckt sich auf die Beschichtung von Glas, Keramik und Metallen in Lösung, wobei als Calciumphosphat-Quelle ein Calciumphosphat-haltiges Glas dient. Die Schichtdicke beträgt etwa 1-2 µm nach 3 Tagen.
4. B.R. Constantz, EP 0 450 939 A2 (eingereicht 03.04.91), *"Improvements in hydroxyapatite coating of substrates*". Dieses Verfahren beschreibt die Beschichtung von Ti, Co, Ta und deren Legierungen sowie von Polyethylen ohne Vorbehandlung durch Tauchen in wässrige Lösung von Calcium und Phosphat in Gegenwart von Ammoniumacetat als Puffer (pH 7.4). Schichtdicken 2-40 µm.
5. K. de Groot, WO 95/13101 (PCT) (eingereicht 08.11.94), "*A method of applying a coating of a bioactive material to implants*". Dieses Verfahren beschreibt die Beschichtung von Titan, Ti6Al4V und Edelstahl ohne Vorbehandlung mit wässriger Calciumphosphat-Lösung. Typische Schichtdicken 1-10 µm.
6. P. Li, US 6 139 585 (eingereicht 11.03.98), "*Bioactive ceramic coating and method*". Dieses Verfahren beschreibt die Beschichtung von Glas, Keramik und Metalle (spezifiziert in nachfolgendem Anspruch auf Titan, Titanlegierungen, Co-Cr-Legierungen; Ta und Ta-Legierungen, Edelstahl) ohne Vorbehandlung des Substrats. Schichtdicken 0.005-50 µm in 1-7 Tagen.
7. S. Sarangapani and P.D. Calvert, US 6 129 928 (eingereicht 04.09.98), *"Biomimetic calcium phosphate implant coatings and methods for making the same*". Dieser Verfahren beschreibt die Beschichtung von Ti, Co, Cr, Nb, Ta und deren Legierungen aus wässriger Calciumphosphat-Lösung nach Ätzen mit NaOH und H₂O₂. Typische Schichtdicken 20-30 µm im 5 Tagen. Nachbehandlung mit einem Hydrogel-Polymer.
8. Y. Yokigawa, et al., US 6 153 266 (eingereicht 07.12.98), *"Method for producing caicium phosphate coating film*". Dieses Verfahren beschreibt ein Tauchverfahren, bei dem zuerst in eine Phosphat-Lösung und anschließend in eine Calcium-Lösung getaucht wird. Als dritte Lösung dient eine Calciumphosphat-Lösung. Als Substrate werden Polymere und Fasern beschrieben.
9. P.J.F. Layrolle and K. de Groot, EP 0 987 031 A1 (eingereicht 12.07.99), "*Method for coating medical implants*". Dieses Verfahren beschreibt die Beschichtung von Edelstahl, Ti, Ni, Co, Cr, Nb, Mo, Zr, Ta und deren Legierungen und von Keramiken aus wässriger Calciumphosphat-Lösung unter CO₂-Atmosphäre. Typische Schichtdicken 1-5 µm nach einem Tag.

### Abgrenzung des hier beschriebenen Erzeugnisses/Verfahrens von den vorstehenden Schutzrechten:

1. Die Formgedächtnislegierung Nitinol kann formal zwar als Titan-Legierung aufgefasst werden, jedoch erwähnt keine der vorstehenden Anmeldungen den Formgedächtniseffekt oder Nickel als Bestandteil einer Ti-Legierung. Eine solche Legierung wäre aufgrund der allergenen Wirkung des Nickels auch nicht sinnvoll, es sei denn, dass ausdrücklich auf den Formgedächtniseffekt abgezielt wird. Dies wird aber in keinem der Verfahren erwähnt. Weiterhin weist die intermetallische Phase NiTi eine völlig neue Qualität (nämlich den Formgedächtniseffekt) auf, d.h. es ist nicht "nur" eine Legierung von Titan, z.B. zur Erhöhung der mechanischen Festigkeit (wie beispielsweise Ti6Al4V = Ti mit 6 % A1 und 4 % V). Zudem hat auch die Beschichtung eine völlig neue Funktion, die darin besteht, die Freisetzung von Nickel zu unterdrücken.
2. Die in den Schutzrechten beschriebenen Verfahren eignen sich nicht zur Beschichtung von Nitinol (und anderen Ni-Ti-Legierungen). Im Rahmen der Erfindung wird eine chemische Vorbehandlung des Nitinols vorgenommen, um eine Calciumphosphat-Abscheidung auf dem Substrat sichergestellt. Das hier vorgeschlagene Verfahren umfasst insbesondere die Ätzung zunächst mit H₂O₂, gefolgt von KOH oder Ca(OH)₂ (s.u.). Die Schutzrechte 1, 3, 4, 5, 6, 8 umfassen keine Vorbehandlung. Schutzrecht 2 umfasst eine Ätzung mit NaOH oder KOH, gefolgt von Erhitzen auf 400 bis 800°C. Diese Behandlung führt bei Nitinol nicht zur Abscheidung einer stabilen Schicht. Schutzrecht 9 beschreibt eine Ätzung mit Säure im Gemisch mit H₂O₂. Dies führt zum Verlust der Superelastizität des Materials. Schutzrecht 7 umfasst die Ätzung mit NaOH, gefolgt von H₂O₂. Dies gibt signifikant schlechter anhaftende Schichten als das erfindungsgemäße Verfahren.
3. Das im nachfolgenden beschriebene Verfahren wurde als optimal für die Beschichtung von Nitinol gefunden. Die Beschichtung ist in der Lage, die mehrprozentige Volumenänderung und die Geometrieänderung beim Durchlaufen des Formgedächtniseffekts unversehrt zu überstehen. Es ist schnell (hohe Abscheidungsrate), einfach (keine weiteren Aufbauten erforderlich) und kostengünstig.

### Einsatzmöglichkeiten und Vorteile der Beschichtung

1) Unterdrückung der Nickelfreisetzung durch Immobilisierung von freiwerdenden Nickel-Ionen in der Deckschicht.
2) Eröffnung neuer Einsatzbereiche, vor allem im chirurgischen Bereich, durch Eliminierung der allergenen Wirkung des enthaltenen Nickels.
3) Verbesserung der Biokompatibilität von Nitinol und anderen Nickel-Titan-Legierungen für alle medizinischen Einsatzgebiete durch Überdeckung mit Calciumphosphat.
4) Verbesserung des Kurz- und Langzeitverhaltens von derzeit verwendeten Implantaten (z.B. Stents, chirurgische Implantate, orthodontische Drähte) unter Erhalt des erwünschten Formgedächtniseffekts.
5) Selbstheilung der Calciumphosphat-Schicht auch nach mechanischer Beschädigung durch Abscheidung von Calciumphosphat aus den übersättigten Körperflüssigkeiten Blutserum und Speichel auf der als Substrat dienenden Oberfläche.
6) Verbesserung des kosmetischen Effekts bei orthodontischen Drähten durch Aufbringen einer weißen Beschichtung aus Calciumphosphat.
7) Besser definiertes Calciumphosphat, z.B. ist die Abscheidung von Carbonatapatit (ähnlich dem Knochenmineral) möglich.
8) Innenbeschichtung poröser Werkstücke ist möglich.
9) Keine thermische Belastung des Werkstücks.
10) Inkorporation biologischer Moleküle ist leicht möglich.
11) Einfaches und kostengünstiges Verfahren.

### Beispiel für die Beschichtung

Eine Platte aus Nitinol (5·30·0.2 mm³) wird mit Aceton (10 min) und dann mit Ethanol (10 min) gereinigt und entfettet. Anschließend wird sie mit destilliertem Wasser gespült. Die Platte wird für 60 min in 30 % H₂O₂-Lösung gekocht und anschließend mit destilliertem Wasser gespült. Die Ätzung erfolgt mit 4 M KOH-Lösung für 30 min bei 120°C. Danach wird mehrfach mit Wasser abgespült. Nach dieser Behandlung weist die Probe eine hohe Oberflächenrauhigkeit im µm-Bereich auf (nachgewiesen durch Rasterelektronenmikroskopie, REM). Zur Abscheidung von Calciumphosphat wird die Probe bei 20°C in eine wässrige Lösung mit den folgenden Ionen eingetaucht: 3.1 mM K⁺, 3.0 mM Ca²⁺, 1.8 mM HPO₄²⁻, 3.0 mM NO₃⁻. Der pH-Wert wurde zuvor mit KOH-Lösung auf 7.0 eingestellt. Nach 24 Stunden betrug die Dicke der abgeschiedenen Schicht 10 µm. Die Schichtfestigkeit kann signifikant durch Herausnehmen, Trocknen und erneutes Einlegen in die Calciumphosphat-Lösung erhöht werden (Erhöhung der Verdichtung durch Kristallisation innerhalb der porösen Schicht).

Alternativ kann das Ätzmittel KOH durch Ca(OH)₂-Lösung (gesättigt, 170°C, 20 Stunden, geschlossenes Gefäß) ersetzt werden.

Der Selbstheilungseffekt kann leicht durch absichtliches mechanisches Beschädigen der Schicht, gefolgt von erneuter Immersion in Calciumphosphat-Lösung, demonstriert werden.

Die Abbildungen 1 und 2 zeigen typische Calciumphosphat-Schichten. Es sei betont, dass die nicht völlig kompakte Schicht wichtig ist, um die Dehnung des Metalls kompensieren zu können.

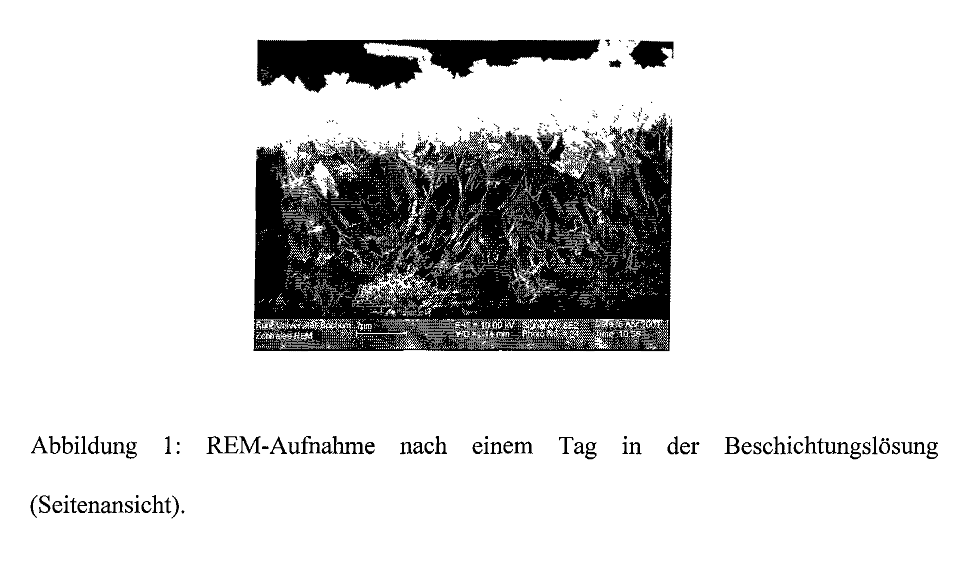

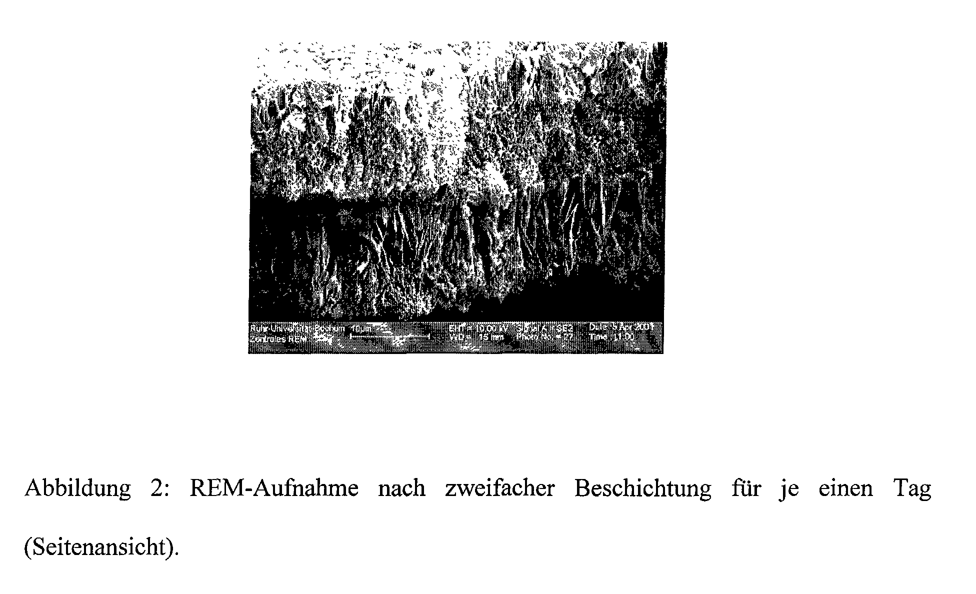

## Patentansprüche

1. Erzeugnis für die Biomedizin mit einem eine Nickel-Titanlegierung enthaltenden Substrat und mindestens einer, durch Eintauchen in Calciumphosphat-Lösung auf das Substrat aufgebrachten, mindestens ein Calciumphosphat enthaltenden Schicht.

2. Erzeugnis nach Anspruch 1, bei dem die Legierung auch andere Metalle als Nickel und Titan enthält.

3. Erzeugnis nach Anspruch 1 oder 2, bei dem die Legierung eine Formgedächtnislegierung ist.

4. Erzeugnis nach einem der Ansprüche 1 bis 3, bei dem das Substrat angeätzt und/oder oxidiert ist.

5. Erzeugnis nach einem der Ansprüche 1 bis 4, bei dem das Substrat eine Oxide umfassende Deckschicht enthält, die als Komponente TiO₂ und/oder CaO und/oder K₂O umfaßt.

6. Erzeugnis nach einem der Ansprüche 1 bis 5, bei dem die Schicht weitere anorganische Ionen enthält, die im Blutplasma oder im Speichel enthalten sind.

7. Erzeugnis nach einem der Ansprüche 1 bis 6, bei dem die Schicht biologisch aktive Komponenten enthält.

8. Erzeugnis nach einem der Ansprüche 1 bis 7, bei dem die Schicht organische Verbindungen enthält, die für die Morphologie der Schicht bestimmend sind.

9. Erzeugnis nach einem der Ansprüche 1 bis 8, das ein Implantat, eine Prothese, ein Operationshilfsmittel oder ein biomedizinisches Drahtmaterial ist.

10. Erzeugnis nach einem der Ansprüche 1 bis 9, das einen biomedizinischen Wirkstoff enthält, der entweder in dem Substrat unter der wirkstoffdurchlässigen Schicht oder in der wirkstoffdurchlässigen Schicht enthalten ist.

11. Erzeugnis nach einem der Ansprüche 1 bis 10, das ein orthodontischer Draht mit einer hell gefärbten Schicht ist.

12. Verfahren, geeignet zur Herstellung eines Erzeugnisses nach einem der Ansprüche 1 bis 11, bei dem durch Eintauchen eines eine Nickel-Titan-Legierung umfassenden Substrats in eine Calciumphosphat-Lösung mindestens eine ein Calciumphosphat umfassende Schicht auf dem Substrat aufgebracht wird.

13. Verfahren nach Anspruch 12, bei dem die Legierung auch andere Metalle als Nickel und Titan enthält.

14. Verfahren nach Anspruch 12 oder 13, bei dem die Legierung eine Formgedächtnislegierung ist.

15. Verfahren nach einem der Ansprüche 12 bis 14, bei dem das Substrat vor dem Aufbringen der Schicht chemisch vorbehandelt wird, um die Anlagerung des Calciumphosphates an dem Substrat zu verbessern.

16. Verfahren nach Anspruch 15, bei dem das Substrat durch Anätzen und/oder Oxidieren vorbehandelt wird.

17. Verfahren nach einem der Ansprüche 12 bis 16, bei dem das Substrat zunächst mit H₂O₂ und anschließend mit einer Base vorbehandelt wird.

18. Verfahren nach Anspruch 17, bei dem das Substrat für etwa 60 min in etwa 30 % H₂O₂-Lösung gekocht wird.

19. Verfahren nach Anspruch 17 oder 18, bei dem das Substrat mit KOH oder Ca(OH)₂ vorbehandelt wird.

20. Verfahren nach Anspruch 19, bei dem das Substrat für etwa 30 min bei etwa 120 °C in etwa 4 M KOH-Lösung oder für etwa 20 Stunden bei etwa 170 °C in gesättigter Ca(OH)₂-Lösung vorbehandelt wird.

21. Verfahren nach einem der Ansprüche 12 bis 22, bei dem weitere anorganische Ionen, die im Blutplasma oder im Speichel enthalten sind, aus der wäßrigen Lösung in die Schicht eingelagert werden.

22. Verfahren nach einem der Ansprüche 12 bis 21, bei dem biologisch aktive Komponenten aus der wäßrigen Lösung in die Schicht eingelagert werden.

23. Verfahren nach einem der Ansprüche 12 bis 22, bei dem die wäßrige Lösung organische Verbindungen zur Steuerung der Morphologie der Calciumphosphate enthaltenden Schicht enthält.

24. Verfahren nach einem der Ansprüche 12 bis 23, bei dem mehrere Schichten aufgebracht werden.

25. Verfahren nach Anspruch 24, bei dem eine aufgebrachte Schicht getrocknet wird, bevor die nächste Schicht aufgebracht wird.

26. Verfahren nach einem der Ansprüche 12 bis 25, bei dem die Beschaffenheit der Schicht durch Variation der Temperatur der wäßrigen Lösung eingestellt wird.

27. Verfahren nach einem der Ansprüche 12 bis 26, bei dem Implantate, Prothesen, Operationshilfsmittel oder biomedizinische Drähte, die die Legierung zumindest an einer Oberfläche aufweisen, an dieser Oberfläche mit Calciumphosphaten beschichtet werden.

28. Verfahren nach Anspruch 27, bei dem wirkstoffabgebende Implantate, Prothesen, Operationshilfsmittel oder biomedizinische Drähte mit einer wirkstoffdurchlässigen Schicht beschichtet und/oder Implantate, Prothesen, Operationshilfsmittel oder biomedizinische Drähte mit einer wirkstoffabgebenden Schicht beschichtet werden.

29. Verfahren nach einem der Ansprüche 12 bis 28, bei dem orthodontische Drähte mit einer hell gefärbten Schicht beschichtet werden.
